(19) Europäisches Patentamt
European Patent Office
Office européen des brevets

(11) **EP 4 654 206 A1**

(12) **EUROPEAN PATENT APPLICATION**
published in accordance with Art. 153(4) EPC

(43) Date of publication:
**26.11.2025 Bulletin 2025/48**

(21) Application number: **24760291.5**

(22) Date of filing: **19.02.2024**

(51) International Patent Classification (IPC):
***G16B 15/30*** (2019.01)

(52) Cooperative Patent Classification (CPC):
**G16B 15/30**

(86) International application number:
**PCT/JP2024/005667**

(87) International publication number:
**WO 2024/176992 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **21.02.2023 JP 2023025282**

(71) Applicant: **Kagoshima University
Kagoshima-shi, Kagoshima 890-8580 (JP)**

(72) Inventor: **ISHIKAWA Takeshi
Kagoshima-shi, Kagoshima 890-8580 (JP)**

(74) Representative: **v. Bezold & Partner
Patentanwälte - PartG mbB
Ridlerstraße 57
80339 München (DE)**

(54) **INTERMOLECULAR INTERACTION ANALYSIS DEVICE, INTERMOLECULAR INTERACTION ANALYSIS METHOD, AND PROGRAM**

(57) An intermolecular interaction analysis device (1) includes: a molecule locater (12) that determines a location of Molecule B indicated by three-dimensional structure data (22) on Molecule B with respect to a position of Molecule A indicated by three-dimensional structure data (21) on Molecule A; a contact face definer (13) that defines, as a contact face, a face on which an electron density of Molecule A computed from the three-dimensional structure data (21) and an electron density of Molecule B computed from the three-dimensional structure data (22) are equal; and an evaluator (14) that evaluates an attractive interaction between Molecule A and Molecule B on the basis of electrostatic complementarity between Molecule A and Molecule B on the contact face, wherein the complementarity is quantitated from the respective electrostatic potentials of Molecule A and Molecule B computed from the three-dimensional structure data (21) and the three-dimensional structure data (22) respectively.

FIG. 1

EP 4 654 206 A1

## Description

Technical Field

The present disclosure relates to an intermolecular interaction analysis device, an intermolecular interaction analysis method, and a program.

Background Art

Many biological functions in an organism, such as signaling, transport, and metabolism, are controlled by protein-protein interaction (PPI), which is a generic term of interaction between protein molecules. An abnormality in PPI has been revealed to be related to the onset of a disease, or the like. PPI is attracting attention also in development of a pharmaceutical product. An antibody that binds to a target protein through PPI has high affinity and specificity, and thus, is utilized as a pharmaceutical product.

To understand PPI in detail, information on the three-dimensional structure of a protein complex that is a protein-protein complex is useful. To obtain information on the structure of a protein at an atomic-level resolution, X-ray crystallography is usually used. However, crystallizing a protein complex is difficult, and involves lots of time and labor. For this reason, a computer is utilized to predict the three-dimensional structure of a protein complex, as disclosed in Non-Patent Literature 1.

For example, Patent Literature 1 discloses a method of evaluating complementarity between structural units constituting a protein complex, from the shape of the space of a region of association between the structural units, using, as an index of complementarity between the structural units, the spatial volume of the region of association together with the surface area of the region of association between the structural units in the protein complex.

Citation List

Patent Literature

Patent Literature 1: Unexamined Japanese Patent Application Publication No. 2008-135019

Non Patent Literature

Non Patent Literature 1: Sheng-YouHuang, Exploring the potential of global protein-protein docking: an overview and critical assessment of current programs for automatic ab initio docking, Drug Discovery Today, Volume 20, Issue 8, 2015, 969-977

Summary of Invention

Technical Problem

For complementarity between proteins in a protein complex, an electrostatic interaction on a contact face is considered to be important. The method disclosed in Patent Literature 1 does not consider the contribution of an electrostatic interaction in evaluation of the complementarity, and thus, is difficult to describe as being capable of predicting the three-dimensional structure of a protein complex with high accuracy.

The present disclosure has been made in view of the above-described circumstances. An objective of the present disclosure is to provide an intermolecular interaction analysis device, an intermolecular interaction analysis method, and a program that are able to predict, with high accuracy, the three-dimensional structure of a complex of molecules containing a plurality of amino acid residues.

Solution to Problem

An intermolecular interaction analysis device according to a first aspect of the present disclosure includes: a molecule locater that determines a location of a second molecule with respect to a position of a first molecule,

wherein the first molecule contains a plurality of amino acid residues bound via a peptide bond, and is indicated by three-dimensional structure data on the first molecule, and wherein the second molecule contains a plurality of amino acid residues bound via a peptide bond, and is indicated by three-dimensional structure data on the second molecule; a contact face definer that defines, as a contact face, a face on which an electron density of the first molecule computed from the three-dimensional structure data on the first molecule and an electron density of the second molecule computed from the three-dimensional structure data on the second molecule are equal to each other; and

an evaluator that evaluates an attractive interaction between the first molecule and the second molecule on the basis of electrostatic complementarity between the first molecule and the second molecule on the contact face, wherein the electrostatic complementarity is quantitated from an electrostatic potential of the first molecule computed from the three-dimensional structure data on the first molecule and an electrostatic potential of the second molecule computed from the three-dimensional structure data on the second molecule.

The evaluator may evaluate the attractive interaction between the first molecule and the second molecule on the basis of an index indicative of an extent of the contact face and on the basis of a magnitude of the electrostatic complementarity between the first molecule and the second molecule.

The contact face definer may define, as the contact face, a face on which the electron density of the first molecule is equal to or more than a reference value.

The evaluator may quantitate the electrostatic complementarity from:

a value obtained by adding, to a value of the electrostatic potential of the first molecule at each position of the contact face, a first constant for bringing, to 0, a total value of the electrostatic potential of the first molecule on the contact face by shifting the whole electrostatic potential of the first molecule on the contact face, and;

a value obtained by adding, to a value of the electrostatic potential of the second molecule at each position of the contact face, a second constant for bringing, to 0, a total value of the electrostatic potential of the second molecule on the contact face by shifting the whole electrostatic potential of the second molecule on the contact face.

The molecule locater may determine a plurality of locations of the second molecule with respect to the position of the first molecule in accordance with translation of the second molecule in a coordinate system having an x axis, a y axis, and a z axis perpendicular to one other and rotation of the second molecule about the x axis, the y axis, and the z axis as axes of rotation,

the contact face definer may define the contact face for each of the plurality of locations of the second molecule, and the evaluator may evaluate the attractive interaction between the first molecule and the second molecule on each of the contact faces.

The molecule locater may further determine a plurality of locations of the second molecule with respect to the position of the first molecule in accordance with translation of the second molecule in a coordinate system having the x axis, the y axis, and the z axis and rotation of the second molecule about the x axis, the y axis, and the z axis as axes of rotation, using a genetic algorithm, on the basis of a size of the attractive interaction between the first molecule and the second molecule on each of the contact faces evaluated by the evaluator,

the contact face definer may define the contact face for each of the plurality of locations of the second molecule, and the evaluator may evaluate the attractive interaction between the first molecule and the second molecule on each of the contact faces.

The evaluator may select, from the plurality of locations of the second molecule, a location having a large attractive interaction between the first molecule and the second molecule.

The electron density and electrostatic potential of the first molecule and the electron density and electrostatic potential of the second molecule may be computed using a fragment molecular orbital method.

An intermolecular interaction analysis method according to a second aspect of the present disclosure includes:

determining a location of a second molecule with respect to a position of a first molecule,

wherein the first molecule contains a plurality of amino acid residues bound via a peptide bond, and is indicated by three-dimensional structure data on the first molecule, and

wherein the second molecule contains a plurality of amino acid residues bound via a peptide bond, and is indicated by three-dimensional structure data on the second molecule;

defining, as a contact face, a face on which an electron density of the first molecule computed from the three-dimensional structure data on the first molecule and an electron density of the second molecule computed from the three-dimensional structure data on the second molecule are equal to each other; and

evaluating an attractive interaction between the first molecule and the second molecule on the basis of electrostatic complementarity between the first molecule and the second molecule on the contact face, wherein the electrostatic complementarity is quantitated from an electrostatic potential of the first molecule computed from the three-dimensional structure data on the first molecule and an electrostatic potential of the second molecule computed from the three-dimensional structure data on the second molecule.

A program according to a third aspect of the present disclosure is a program that allows a computer to function as:

a molecule locater that determines a location of a second molecule with respect to a position of a first molecule,

wherein the first molecule contains a plurality of amino acid residues bound via a peptide bond, and is indicated by three-dimensional structure data on the first molecule, and
wherein the second molecule contains a plurality of amino acid residues bound via a peptide bond, and is indicated by three-dimensional structure data on the second molecule;

a contact face definer that defines, as a contact face, a face on which an electron density of the first molecule computed from the three-dimensional structure data on the first molecule and an electron density of the second molecule computed from the three-dimensional structure data on the second molecule are equal to each other; and
an evaluator that evaluates an attractive interaction between the first molecule and the second molecule on the basis of electrostatic complementarity between the first molecule and the second molecule on the contact face, wherein the electrostatic complementarity is quantitated from an electrostatic potential of the first molecule computed from the three-dimensional structure data on the first molecule and an electrostatic potential of the second molecule computed from the three-dimensional structure data on the second molecule.

Advantageous Effects of Invention

The present disclosure can predict, with high accuracy, the three-dimensional structure of a complex of molecules containing a plurality of amino acid residues.

Brief Description of Drawings

FIG. 1 is a diagram illustrating the configuration of an intermolecular interaction analysis device according to an embodiment of the present disclosure;
FIG. 2 is a diagram illustrating the computation range of an electron density and an electrostatic potential and the concept of three-dimensional grid data;
FIG. 3A is a diagram illustrating the directions of translation of Molecule B;
FIG. 3B is a diagram illustrating the axes of rotation of Molecule B;
FIG. 4 is a diagram illustrating the location of Molecule B with respect to the position of Molecule A;
FIG. 5 is a diagram illustrating a plurality of locations of Molecule B with respect to the position of Molecule A;
FIG. 6 is a block diagram illustrating the hardware configuration of the intermolecular interaction analysis device in FIG. 1;
FIG. 7 is a diagram illustrating a flow chart of attractive interaction evaluation processing by the intermolecular interaction analysis device in FIG. 6;
FIG. 8 is a diagram illustrating a flow chart of three-dimensional structure prediction processing of a complex by the intermolecular interaction analysis device in FIG. 6; and
FIG. 9 is a diagram illustrating a flow chart of search processing by a genetic algorithm in three-dimensional structure prediction processing of a complex.

Description of Embodiments

An embodiment according to the present disclosure is described below with reference to the drawings. In the drawings, the same reference sign is used for the same or equivalent part. In this regard, the present disclosure is not limited by the below-described embodiment and the drawings. Note that, in the below-described embodiment, the expression, "have", "include", or "contain" encompasses the meaning of "composed of" or "be constituted by".
An embodiment according to the present disclosure is described. An intermolecular interaction analysis device as an information processing device according to the present embodiment analyzes intermolecular interaction between Molecule A (a first molecule) and Molecule B (a second molecule), and predicts the three-dimensional structure of a complex including Molecule A and Molecule B. Molecule A and Molecule B each contain a plurality of amino acid residues bound via a peptide bond. Examples of Molecule A and Molecule B include a protein, peptide, and antibody. Molecule A and Molecule B may be a molecule that contains a plurality of amino acid strands bound via a bond other than a peptide bond, for example, via a disulfide bond, as an antibody does. The intermolecular interaction encompasses an electrostatic interaction, van der Waals force, dipole-dipole interaction, dispersion force, hydrogen bonding, hydrophobic interaction, and the like that act between Molecule A and Molecule B.

An intermolecular interaction analysis device according to the present embodiment is built in a computer. That is, the intermolecular interaction analysis device is an information processing device in which information processing by software is specifically achieved, using a hardware resource.

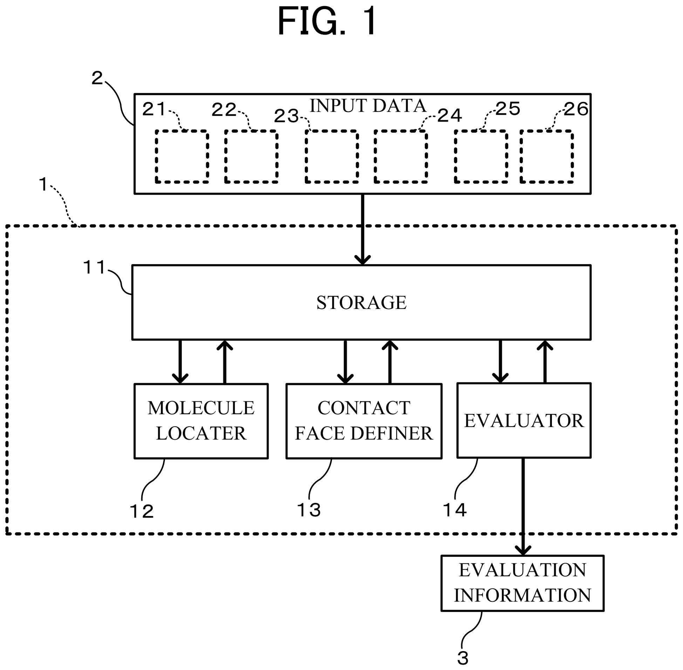

As illustrated in FIG. 1, input data 2 is inputted into an intermolecular interaction analysis device 1. The input data 2 contains three-dimensional structure data 21 on Molecule A, three-dimensional structure data 22 on Molecule B, electron density data 23 on Molecule A, electron density data 24 on Molecule B, electrostatic potential data 25 on Molecule A, and electrostatic potential data 26 on Molecule B.

The three-dimensional structure data 21 is information indicative of the position of each of the atoms constituting Molecule A in a three-dimensional space. The three-dimensional structure data 21 is specifically coordinate data in the atoms constituting Molecule A, as determined by X-ray crystallography, NMR, or the like. The coordinate data refers to, for example, coordinates (x, y, z) indicative of a position in a coordinate system having the x axis, the y axis, and the z axis that are perpendicular to one another. The three-dimensional structure data 21 can be obtained from Protein Data Bank (PDB) or the like. In a case where coordinate data obtained from PDB is used as the three-dimensional structure data 21, water molecules may be deleted from the coordinate data, the three-dimensional structure may be supplemented with a hydrogen atom, and the N terminus and the C terminus may each be capped with $-COCH_3$, $-NHCH_3$, or the like. Alternatively, coordinate data on a three-dimensional structure, optimized using a known empirical parameter (force field), may be used as the three-dimensional structure data 21. The three-dimensional structure data 22 is the same as the three-dimensional structure data 21.

The electron density data 23 and the electrostatic potential data 25 are data respectively indicative of the electron density and electrostatic potential of Molecule A. The electron density data 24 and the electrostatic potential data 26 are data respectively indicative of the electron density and electrostatic potential of Molecule B. The electron density and the electrostatic potential can be computed utilizing a known force field, or can also be computed using *ab initio* calculation. In the present embodiment, a case where the electron density and the electrostatic potential are computed using the fragment molecular orbital (FMO) method, which is *ab initio* calculation, is described.

The FMO method is a method in which a macromolecular system, such as a protein or a polymer, is divided into small fragments in units of tens of atoms, and computation based on a non-empirical molecular orbital method is performed on the single fragments and the fragment pairs over and over again, whereby the total energy of the molecule, the interfragment interaction energy (IFIE), and the like are computed. For the FMO method, a known software program, such as PAICS, can be used.

Here, computation of an electron density by the FMO method is described, taking a protein as an example. First, a protein is separated into fragments according to amino acid residues, whereby the protein is divided into small fragments. For computation for single fragments and fragment pairs, the electrostatic potential needs to be considered on the basis of the electron densities of the surrounding fragments, and thus, monomer self consistent charge (monomer SCC) computation is performed to determine the electron density of each fragment. In the monomer SCC computation, the initial electron density is determined, and computation is performed on each fragment in the presence of an electrostatic potential according to this initial electron density. Upon completion of computation related to all the fragments, a new electron density different from the previous initial electron density is obtained. Subsequently, the same computation is performed using this new electron density as an initial electron density. This computation is repeated to obtain a self-consistent electron density, which is used as the final electron density. Then, considering an electrostatic potential produced by this final electron density, computation for the single fragment and computation for the fragment pair are performed. The property of the whole is finally computed only from the results of computation for the single fragment and computation for the fragment pair. The total energy is determined as illustrated in Formula 1, using the energy ($E_I$) of the single fragment and the energy ($E_{IJ}$) of the fragment pair.

[Math. 1]

$$E_{total} = \sum_{I<J} E_{IJ} - (N-2) \sum_I E_I$$

(Formula 1)

In Formula 1, a replacement is made to give Formula 2 below.
[Math. 2]

$$E_{total} = \sum_{I} E_I + \sum_{I<J} \Delta E_{IJ}$$

(Formula 2)

The first term in the right side in Formula 2 is the energy of a fragment, obtained by removing the electrostatic potential derived from another fragment. The second term in the right side represents IFIE, which is an interfragment interaction energy.

The electron densities of Molecule A and Molecule B at position r are expressed as $\rho^A(r)$ and $\rho^B(r)$ respectively, and the electrostatic potentials of Molecule A and Molecule B at position r are expressed as $\varphi^A(r)$ and $\varphi^B(r)$ respectively. In the FMO method, $\rho^A(r)$ is computed as described below.
[Math. 3]

$$\rho^A(r) = \sum_{I} \rho_I(r) + \sum_{I<J} \Delta\rho_{IJ}(r)$$

(Formula 3)

Here, I and J are fragment numbers, and $\rho_I(r)$ is a value of the electron density of the Ith fragment at position r. $\Delta\rho_{IJ}(r)$ is a correction value used for the electron density owing to a fragment pair of the Ith fragment and the Jth fragment. $\rho^B(r)$ is also computed as described below in the same manner.
[Math. 4]

$$\rho^B(r) = \sum_{I} \rho_I(r) + \sum_{I<J} \Delta\rho_{IJ}(r)$$

(Formula 4)

In the FMO method, $\varphi^A(r)$ is computed as described below.
[Math. 5]

$$\phi^A(r) = \sum_{I} \phi_I(r) + \sum_{I<J} \Delta\phi_{IJ}(r)$$

(Formula 5)

Here, I and J are fragment numbers, and $\varphi_I(r)$ is a value of the electron density of the Ith fragment at position r. $\Delta\varphi_{IJ}(r)$ is a correction value to the electrostatic potential, due to a fragment pair of the Ith fragment and the Jth fragment. $\varphi^B(r)$ is also computed as described below in the same manner.
[Math. 6]

$$\phi^B(r) = \sum_{I} \phi_I(r) + \sum_{I<J} \Delta\phi_{IJ}(r)$$

(Formula 6)

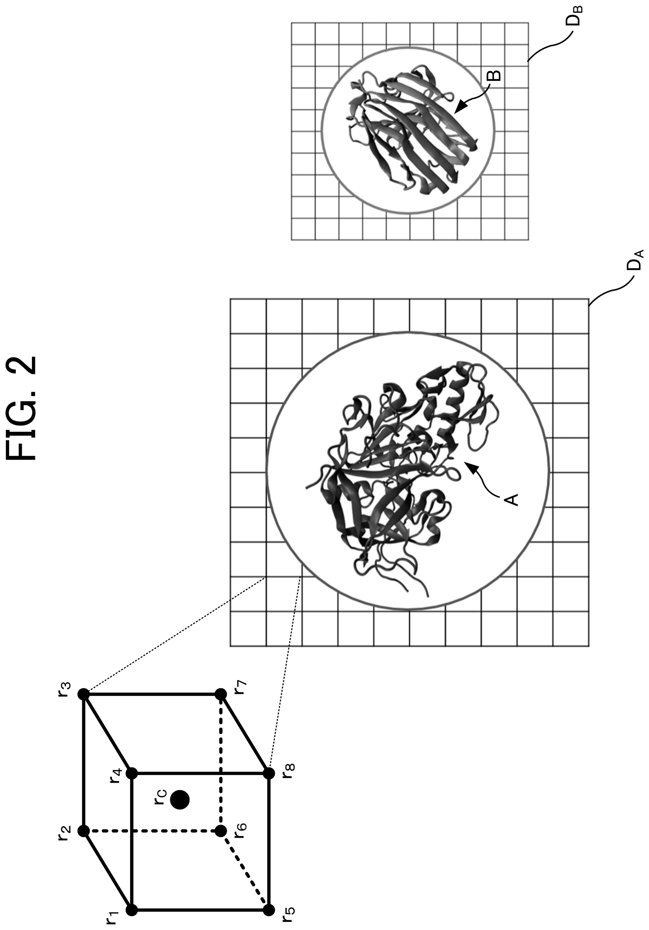

For example, the electron density and the electrostatic potential are computed using three-dimensional grid data. First, to Molecule A illustrated in FIG. 2, a range $D_A$ having grid cubes set out therein is set. The range $D_A$ is set using an arbitrary method so as to contain all the amino acid residues that can contribute to interaction between Molecule A and Molecule B on the surface of Molecule A. For example, the range $D_A$ is a range including all of the distance L from the surface of Molecule A. The distance L is suitably set, considering the size of Molecule A, and the like. The distance L is, for example, 12 Å. Subsequently, the range D is sectioned by grid cubes. The length of a side of the grid cube is suitably set, and is, for example, 0.3 Å. As illustrated in FIG. 2, in a case where attention is paid to one grid cube including eight points, $r_1$ to $r_8$, the electron densities and the electrostatic potentials at $r_1$ to $r_8$ are computed in relation to Molecule A. The electron density of the grid cube computed in relation to Molecule A is obtained as a value at the central point $r_c$ of the grid cube, the value being the average of the electron densities at $r_1$ to $r_8$. In the same manner, the electrostatic potential of the grid cube computed for Molecule A is obtained as a value at the central point $r_c$ of the grid cube, the value being the average of the electrostatic potentials at $r_1$ to $r_8$. In addition, to Molecule B illustrated in FIG. 2, a range $D_B$ having grid cubes set out therein is set in the same manner as to Molecule A, and the electron density and electrostatic potential of Molecule B are computed.

The intermolecular interaction analysis device 1 performs computation on input data 2, and outputs evaluation information 3 related to intermolecular interaction between Molecule A and Molecule B. Evaluation information 3 is, for example, an index indicative of the extent of a contact face between Molecule A and Molecule B; information indicative of electrostatic complementarity; a value of an evaluation function related to attractive interaction; and three-dimensional structure data indicative of a complex of Molecule A and Molecule B that interact via the contact face corresponding to a value of the evaluation function.

The configuration of the intermolecular interaction analysis device 1 is described in more detail. As illustrated in FIG. 1, the intermolecular interaction analysis device 1 includes a storage 11, a molecule locater 12, a contact face definer 13, and an evaluator 14.

The storage 11 stores various data outputted from the input data 2 and besides, the molecule locater 12, the contact face definer 13, and the evaluator 14. The molecule locater 12, the contact face definer 13, and the evaluator 14 can refer to various data, such as the input data 2 stored in the storage 11.

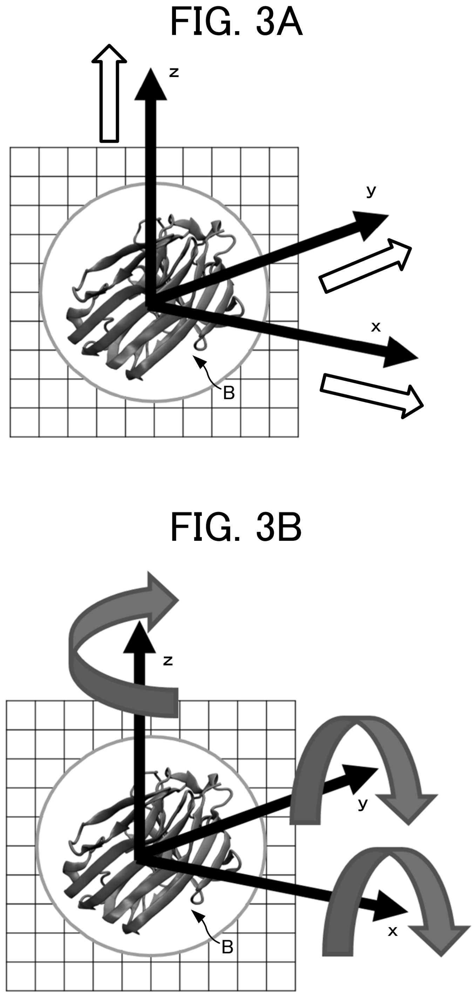

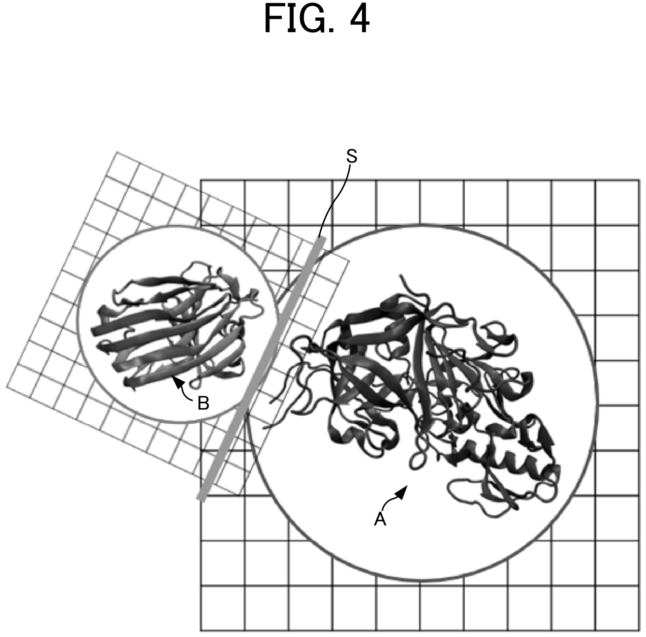

The molecule locater 12 determines the location of Molecule B indicated by the three-dimensional structure data 22 on Molecule B, with respect to the position of Molecule A indicated by the three-dimensional structure data 21 on Molecule A. More specifically, the molecule locater 12 determines the location of Molecule B with respect to the position of Molecule A in accordance with translation of Molecule B in a coordinate system having the x axis, the y axis, and the z axis and rotation of Molecule B about the x axis, the y axis, and the z axis as the axes of rotation. The molecule locater 12 refers to the three-dimensional structure data 21 and the three-dimensional structure data 22 that are stored in the storage 11, and translates Molecule B in the x-axis direction, y-axis direction, and z-axis direction in the same coordinate system as of Molecule A, as illustrated in FIG. 3A. The molecule locater 12 rotates Molecule B about the x axis, the y axis, and the z axis as the axes of rotation in the same coordinate system as of Molecule A, as illustrated in FIG. 3B. The location of Molecule B with respect to the position of Molecule A is thereby determined, as illustrated in FIG. 4. The molecule locater 12 causes the storage 11 to store three-dimensional structure data indicative of the position of Molecule B located with respect to the position of Molecule A (the data is the three-dimensional structure data indicative of the location of Molecule B).

As illustrated in FIG. 4, the contact face definer 13 defines, as a contact face S, a face on which an electron density of Molecule A computed from the three-dimensional structure data 21 and an electron density of Molecule B computed from the three-dimensional structure data 22 on Molecule B are equal to each other. The contact face definer 13 refers to the electron density data 23 and the electron density data 24 stored in the storage 11 to thereby obtain the electron density of Molecule A and the electron density of Molecule B. In a case where the electron density of Molecule A and the electron density of Molecule B are equal in the example illustrated in FIG. 2, the point $r_c$ is the point constituting the contact face S. The contact face definer 13 preferably defines, as the contact face S, a face on which the electron density of Molecule A is equal to or more than the reference value $\rho_c$. $\rho_c$ is, for example, $10^{-4}$ atomic units (a.u.) or more. The contact face definer 13 causes the storage 11 to store, as data indicative of the contact face S, the coordinates of the point $r_c$ at which the electron density of Molecule A and the electron density of Molecule B are equal.

The evaluator 14 evaluates an attractive interaction between Molecule A and Molecule B on the basis of electrostatic complementarity between Molecule A and Molecule B on the contact face S, wherein the electrostatic complementarity is quantitated from an electrostatic potential of Molecule A computed from the three-dimensional structure data 21 on Molecule A and an electrostatic potential of Molecule B computed from the three-dimensional structure data 22 on Molecule B. The evaluation of the attractive interaction by the evaluator 14 is described in detail below.

The evaluator 14 evaluates an attractive interaction between Molecule A and Molecule B on the basis of an index indicative of the extent of the contact face S and on the basis of the magnitude of the electrostatic complementarity between Molecule A and Molecule B. The position of the ith grid cube constituting the contact face S is assumed to be $r_i$. The total value $\rho_{sum}$ of the electron densities of Molecule A on the contact face S can be computed as follows.
[Math. 7]

$$\rho_{sum} = \sum_i \rho^A(r_i)$$

(Formula 7)

$\rho_{sum}$ is the sum of the electron densities of Molecule A at all the grid cubes constituting the contact face S. The electron density of Molecule B on the contact face S can be computed in the same manner. The contact face S is defined as a face including the point $r_c$ at which the electron density of Molecule A and the electron density of Molecule B are equal, and hence, the electron density of Molecule B results in the same value as $\rho_{sum}$ computed above. $\rho_{sum}$ is an index indicative of the size of an overlap between Molecule A and Molecule B, that is, the extent of the contact face S.

The evaluator 14 quantitates the electrostatic complementarity from: a value obtained by adding, to the value of the electrostatic potential of Molecule A at each position of the contact face S, a constant $C_A$ (a first constant) for bringing, to 0, the total value of the electrostatic potential of Molecule A on the contact face S by shifting the whole electrostatic potential of Molecule A on the contact face S, and; a value obtained by adding, to the value of the electrostatic potential of Molecule B at each position of the contact face S, a constant $C_B$ (a second constant) for bringing, to 0, the total value of the electrostatic potential of Molecule B on the contact face S by shifting the whole electrostatic potential of Molecule B on the contact face S.

Bringing, to 0, the total value of the electrostatic potential of Molecule A on the contact face S by shifting the whole electrostatic potential of Molecule A on the contact face S is defined as "neutralization". In a case where an electrostatic potential is computed in accordance with three-dimensional grid data, for example, in a case where $C_A$ is added to $\varphi^A(r_i)$ to bring about the neutralization, assuming that the number of grid cubes constituting the contact face S is N, the following formula holds true.

[Math. 8]

$$\sum_{i=1}^{N} (\phi^A(r_i) + C_A) = 0 \qquad \text{(Formula 8)}$$

$C_A$ that satisfies Formula 8 is determined as follows.

[Math. 9]

$$\sum_{i=1}^{N} \phi^A(r_i) + \sum_{i=1}^{N} C_A = 0 \qquad \text{(Formula 9)}$$

$$\sum_{i=1}^{N} \phi^A(r_i) + NC_A = 0 \qquad \text{(Formula 10)}$$

$$NC_A = -\sum_{i=1}^{N} \phi^A(r_i) \qquad \text{(Formula 11)}$$

$$C_A = -\frac{\sum_{i=0}^{N} \phi^A(r_i)}{N} \qquad \text{(Formula 12)}$$

As represented by Formula 12, $C_A$ is a value obtained by dividing the total value of the electrostatic potential of Molecule A on the contact face S by the number of grid cubes in the contact face S, and making the value minus. In the same manner, $C_B$ is a value obtained by dividing the total value of the electrostatic potential of Molecule B on the contact face S by the number of grid cubes in the contact face S, and making the value minus. The neutralization by the $C_A$ and the $C_B$ is more specifically described as follows: the evaluator 14 quantitates electrostatic complementarity from a value obtained by adding the $C_A$ to the value of the electrostatic potential of Molecule A at each position $r_i$ on the contact face S and a value obtained by adding the $C_B$ to the value of the electrostatic potential of Molecule B at each position $r_i$ on the contact face S. That is, the neutralized electrostatic potential $\varphi'^A(r_i)$ of Molecule A and the neutralized electrostatic potential $\varphi'^B(r_i)$ of Molecule B are represented by the following formulas.

$$\varphi'^A(r_i) = \varphi^A(r_i) + C_A \quad \text{(Formula 13)}$$

$$\varphi'^B(r_i) = \varphi^B(r_i) + C_B \quad \text{(Formula 14)}$$

To quantitate the electrostatic complementarity between Molecule A and Molecule B on the contact face S, the neutralized electrostatic potentials $\varphi'^A(r)$ and $\varphi'^B(r)$ are used to define $\varphi^+$ and $\varphi^-$ as follows.
[Math. 10]

$$\phi^+ = \sum_i \left| \phi'^A(r) + \phi'^B(r) \right|$$

(in a case where $\varphi'^A(r_i)$ and $\varphi'^B(r_i)$ have the same signs as each other)   (Formula 15)

$$\phi^- = \sum_i \left| \phi'^A(r) - \phi'^B(r) \right|$$

(in a case where $\varphi'^A(r_i)$ and $\varphi'^B(r_i)$ have opposite signs to each other)   (Formula 16)

Furthermore, $\varphi^+$ and $\varphi^-$ are used to define $D^-$ as follows.

$$D^- = \varphi^- - \varphi^+ \quad \text{(Formula 17)}$$

In a case where the electrostatic potentials of Molecule A and Molecule B have opposite signs to each other to a higher degree at the same position on the contact face S, the value of $D^-$ is larger. Thus, $D^-$ is an index indicative of electrostatic complementarity between Molecule A and Molecule B on the contact face S.

In a case where Molecule A and Molecule B are in contact with each other in a wider range, and where the electrostatic complementarity on the contact face S is higher, presumably, a larger attractive interaction can be obtained to constitute a stable complex. Accordingly, using $\rho_{sum}$ as an index indicative of the extent of the contact face S and $D^-$ as an index indicative of the electrostatic complementarity on the contact face S, the product of $\rho_{sum}$ and $D^-$ can be utilized as an evaluation function indicative of the size of the attractive interaction between Molecule A and Molecule B.

$$\text{Evaluation function} = D^- \times \rho_{sum} \quad \text{(Formula 18)}$$

In this regard, the evaluation function is not limited to the product of $\rho_{sum}$ and $D^-$, and may be, for example, the sum of $\rho_{sum}$ and $D^-$ (linear combination), or $\rho_{sum}$ and $D^-$ may be weighted. In addition, the index indicative of the extent of the contact face S is not limited to $\rho_{sum}$, and may be the area of the contact face S, or may be the number of grid cubes constituting the contact face S, or the like.

The positional relationship between Molecule A and Molecule B, the value of the evaluation function of which relationship is larger, is considered to represent a stable three-dimensional structure of a complex of Molecule A and Molecule B. As illustrated in FIG. 3A and FIG. 3B, the relative position of Molecule B to Molecule A is determined by six degrees of freedom: the translation of Molecule B in the x-axis direction, y-axis direction, and z-axis direction; and the rotation of Molecule B about the x axis, the y axis, and the z axis as the axes of rotation. Accordingly, optimizing the six degrees of freedom makes it possible to predict the three-dimensional structure of a complex of Molecule A and Molecule B, between which the attractive interaction evaluated using an evaluation function is larger.

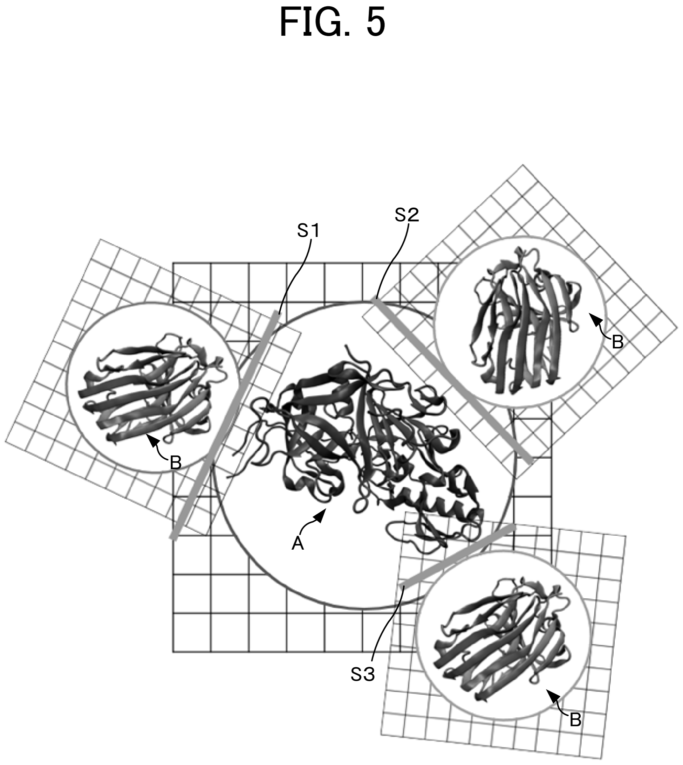

In a case where the three-dimensional structure of a complex of Molecule A and Molecule B is predicted, the molecule locater 12 refers to the three-dimensional structure data 21 and the three-dimensional structure data 22 stored in the storage 11, and determines a plurality of locations of Molecule B with respect to the position of Molecule A from the translation of Molecule B in a coordinate system having the x axis, the y axis, and the z axis and the rotation of Molecule B about the x axis, the y axis, and the z axis as the axes of rotation, for example, as illustrated in FIG. 5. The molecule locater 12 causes the storage 11 to store three-dimensional structure data on Molecule B indicative of each position of Molecule B located with respect to the position of Molecule A.

Subsequently, the contact face definer 13 defines the contact face S related to each of the plurality of locations of Molecule B. Referring to FIG. 5, the contact face definer 13 defines the contact faces S1, S2, and S3, as described above. The evaluator 14 evaluates an attractive interaction between Molecule A and Molecule B on each of the contact faces S1, S2, and S3.

The location of Molecule B with respect to the position of Molecule A represents an optimization problem of six degrees of freedom, as described above. A known method is adopted for the intermolecular interaction analysis device 1 to search for six degrees of freedom to predict the three-dimensional structure of a complex of Molecule A and Molecule B with high accuracy. Examples of an algorithm for the optimization include a genetic algorithm, the Monte Carlo method, and the metaheuristics method.

In a case where six degrees of freedom are searched for through a genetic algorithm, the molecule locater 12 further determines a plurality of locations of Molecule B with respect to the position of Molecule A in accordance with the translation of Molecule B in a coordinate system having the x axis, the y axis, and the z axis and the rotation of Molecule B about the x axis, the y axis, and the z axis as the axes of rotation, using a genetic algorithm, on the basis of the size of the attractive interaction evaluated by the evaluator 14 between Molecule A and Molecule B on each of the contact faces S1, S2, and S3. For example, a genetic algorithm involves randomly generating an $N_1$ number of initial groups of six variables as the values of the vectors in the x-axis direction, y-axis direction, and z-axis direction and the values of rotation vectors in the x-axis direction, y-axis direction, and z-axis direction. The molecule locater 12 determines $N_1$ locations of Molecule B with respect to Molecule A, in which the locations are according to the six variables generated.

As described above, in relation to each of the plurality of locations of Molecule B, the evaluator 14 evaluates an attractive interaction between Molecule A and Molecule B on each of the plurality of contact faces S defined by the contact face definer 13. The molecule locater 12 ranks the locations of Molecule B in the order of magnitude of the attractive interaction, and selects six variables as parents from the higher-ranked locations. In this regard, the molecule locater 12 may regard two locations of Molecule B as duplicate, and delete one of the locations in a case where a difference between the variables at the two locations is smaller than a predetermined value. The molecule locater 12 makes a combination of parents selected, and generates $N_2$ sets of six child variables ($N_1 = 2N_2$) from the parents (crossover). The molecule locater 12 changes part of the generated six variables of a child to an arbitrary value(s) with a probability (a mutation). Furthermore, the molecule locater 12 newly makes, as children, $N_2$ groups of six variables randomly. In relation to the children generated, the molecule locater 12 determines $N_1$ locations of Molecule B with respect to Molecule A, in which the locations are according to the six variables.

The evaluator 14 selects, from the plurality of locations of Molecule B, a location having a large attractive interaction between Molecule A and Molecule B. In a case where, in the above-described genetic algorithm, the number of times when children are generated from a parent is assumed to be the generation number G, the evaluator 14, for example, generates children until G becomes a preset threshold generation number, and causes the storage 11 to store three-dimensional structure data indicative of the three-dimensional structure and the size of the attractive interaction of the structure in a corresponding manner, in which the structure is the structure of a complex of Molecule A and Molecule B at each of the locations of Molecule B with respect to Molecule A, in which the locations are according to the six child variables generated. The molecule locater 12 refers to the storage 11, and selects three-dimensional structure data indicative of the three-dimensional structure of a complex of Molecule A and Molecule B at the locations of Molecule B, at which locations the size of the attractive interaction is higher-ranked.

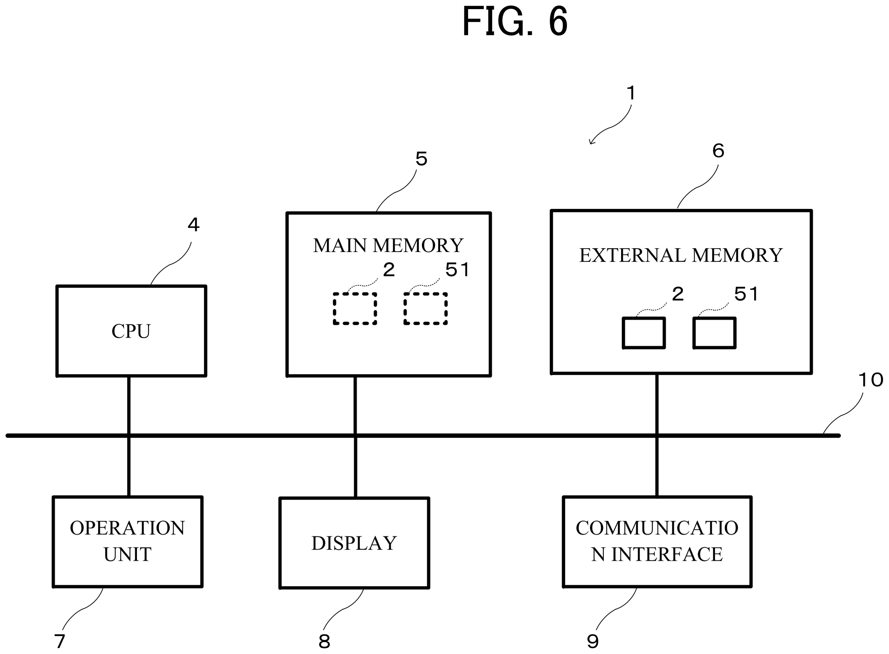

The intermolecular interaction analysis device 1 illustrated in FIG. 1 is actuated, for example, through performance of a software program by a computer having a hardware configuration illustrated in FIG. 6. Specifically, the intermolecular interaction analysis device 1 includes: a central processing unit (CPU) 4 that controls the whole device; a main memory 5 that operates as a workspace of CPU 4, or the like; an external memory 6 that stores the operational program of the CPU 4, and the like; an operation unit 7; a display 8; a communication interface 9; and an internal bus 10 that connects these.

The main memory 5 includes a random access memory (RAM) or the like. Into the main memory 5, input data 2 and a computing program 51 are loaded from an external memory 6. In addition, the main memory 5 is also used as a workspace (temporary data storage area) of the CPU 4.

The external memory 6 includes a nonvolatile memory, such as a flash memory or a hard disk. In the external memory 6, a computing program 51 to be performed by the CPU 4 is preliminarily stored.

The operation unit 7 includes devices, such as a keyboard and a mouse, and an interface device that connects these devices to the internal bus 10.

The display 8 includes a device for display, such as a cathode ray tube (CRT) or a liquid crystal monitor.

The communication interface 9 is an interface that performs data transmission and reception to and from external equipment. Via the communication interface 9, the input data 2 and the like are received, and the evaluation information 3 and the like are transmitted.

Next, attractive interaction evaluation processing is described as an intermolecular interaction analysis method that is performed by operation of an intermolecular interaction analysis device 1 according to the present embodiment.

First, the input data 2 and the computing program 51 are read from the external memory 6 into the main memory 5, and the CPU 4 performs the computing program 51, whereby attractive interaction evaluation processing is performed.

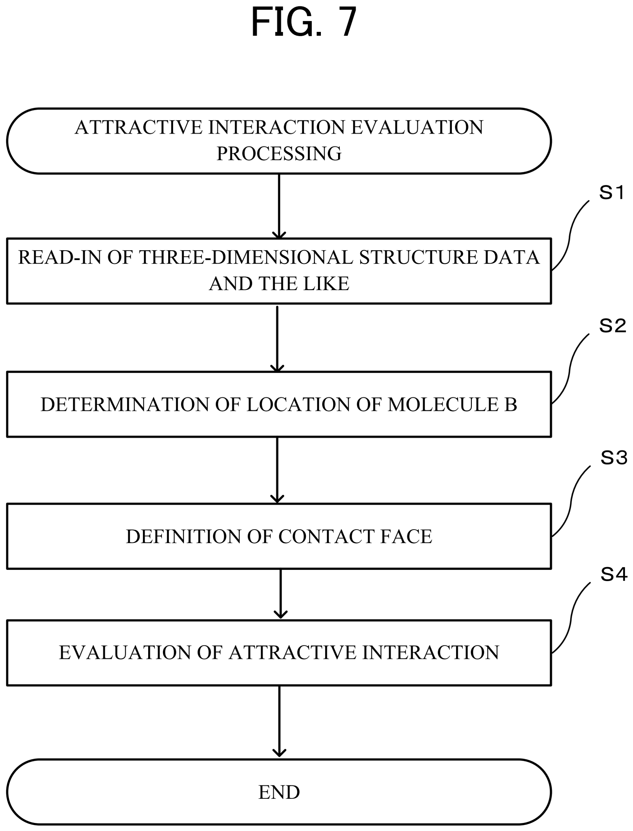

As illustrated in FIG. 7, the intermolecular interaction analysis device 1 reads the three-dimensional structure data 21, the three-dimensional structure data 22, the electron density data 23, the electron density data 24, the electrostatic potential data 25, and the electrostatic potential data 26 (Step S1). Here, the three-dimensional structure data 21, the three-dimensional structure data 22, the electron density data 23, the electron density data 24, the electrostatic potential data 25, and the electrostatic potential data 26 are read from the external memory 6 into the main memory 5.

The molecule locater 12 refers to the three-dimensional structure data 21 and the three-dimensional structure data 22, and determines the location of Molecule B with respect to the position of Molecule A (Step S2). The three-dimensional structure data indicative of the location of Molecule B is stored in the main memory 5 and the external memory 6. Subsequently, the contact face definer 13 refers to the three-dimensional structure data indicative of the location of Molecule B, the electron density data 23, and the electron density data 24, and defines the contact face S (Step S3). The data indicative of the contact face S is stored in the main memory 5 and the external memory 6.

Next, the evaluator 14 refers to the data indicative of the contact face S, the electrostatic potential data 25, and the electrostatic potential data 26, and evaluates an attractive interaction between Molecule A and Molecule B on the contact face S in accordance with an evaluation function (Step S4). The intermolecular interaction analysis device 1 stores the evaluation information 3 in the main memory 5 and the external memory 6, and terminates the attractive interaction evaluation processing.

In this regard, in accordance with the input of the instruction of a user via the operation unit 7, the CPU4 may display, on the display 8, the three-dimensional structure of a complex of Molecule A and Molecule B that interact via the contact face S, in which the structure corresponds to a value of the evaluation function obtained.

Subsequently, three-dimensional structure prediction processing of a complex is described as an intermolecular interaction analysis method that is performed by operation of an intermolecular interaction analysis device 1 according to the present embodiment.

The input data 2 and the computing program 51 are read from the external memory 6 into the main memory 5, and the CPU 4 performs the computing program 51, whereby three-dimensional structure prediction processing of a complex is performed. In this regard, at the start of three-dimensional structure prediction processing of a complex, the above-described G is deemed as the initial value 0.

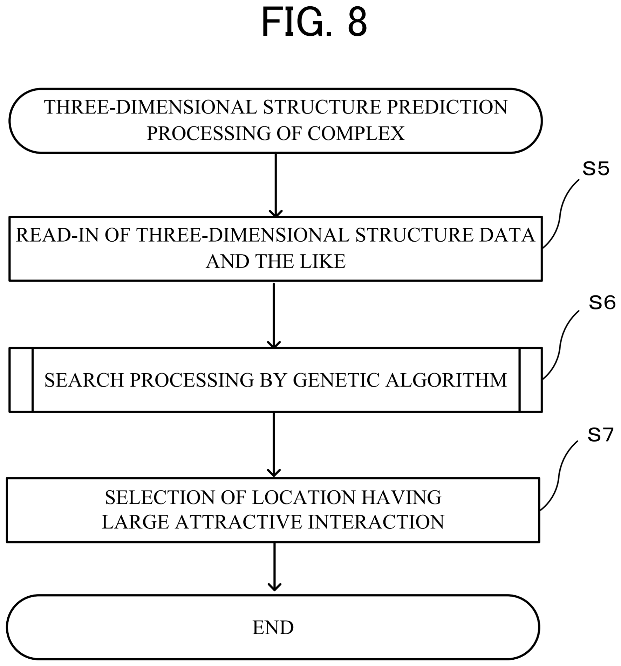

As illustrated in FIG. 8, the intermolecular interaction analysis device 1 reads the three-dimensional structure data 21, the three-dimensional structure data 22, the electron density data 23, the electron density data 24, the electrostatic potential data 25, and the electrostatic potential data 26 (Step S5), in the same manner as in Step S1.

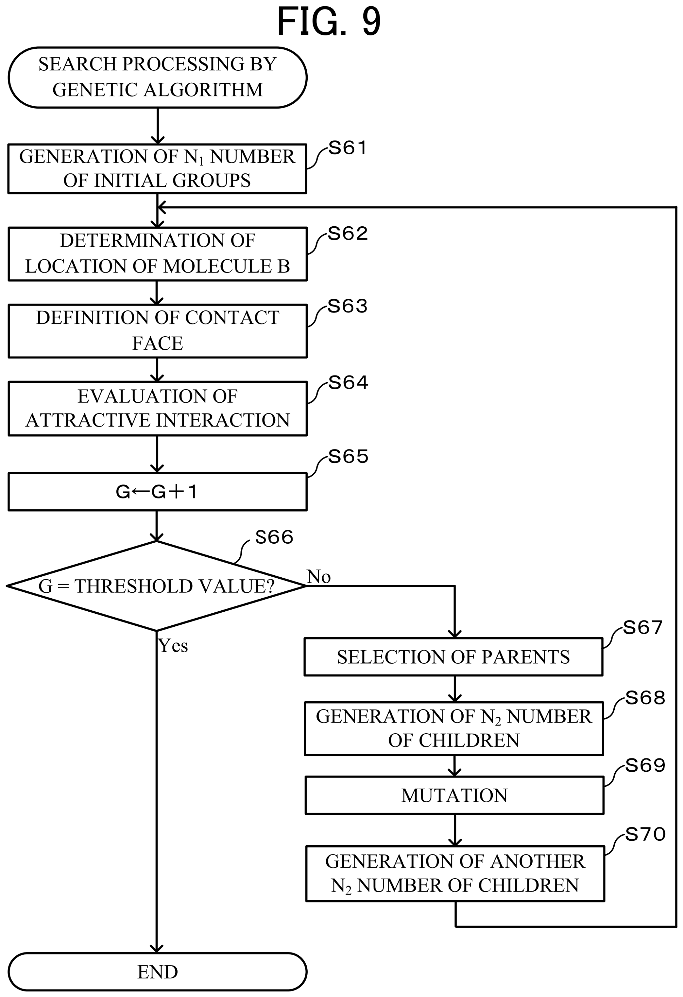

The intermolecular interaction analysis device 1 searches for the location of Molecule B with respect to the position of Molecule A, in accordance with a genetic algorithm (Step S6). With reference to FIG. 9, search processing by a genetic algorithm is described. The molecule locater 12 generates an $N_1$ number of initial groups (Step S61). The molecule locater 12 determines $N_1$ locations of Molecule B with respect to Molecule A (Step S62). The three-dimensional structure data indicative of the location determined of Molecule B is stored in the main memory 5 and the external memory 6. The contact face definer 13 refers to the three-dimensional structure data indicative of the location of Molecule B, the electron density data 23, and the electron density data 24, and defines the contact face S (Step S63). The data indicative of the contact face S is stored in the main memory 5 and the external memory 6.

In relation to each of the plurality of locations of Molecule B, the evaluator 14 evaluates an attractive interaction between Molecule A and Molecule B on each of the plurality of contact faces S defined by the contact face definer 13 (Step S64). Subsequently, the evaluator 14 adds 1 to G (Step S65). The evaluator 14 determines whether G is a threshold value (Step S66). In a case where G is less than the threshold value (Step S66; No), the molecule locater 12 selects six variables as parents from the locations higher-ranked in the attractive interaction (Step S67). Next, the molecule locater 12 generates $N_2$ sets of six child variables from the parents selected (Step S68). Furthermore, the molecule locater 12 produces a mutation, that is, changes part of the six child variables generated to an arbitrary value(s) (Step S69). Furthermore, the molecule locater 12 newly makes, as children, $N_2$ groups of six variables (Step S70). Returning to Step S62, the molecule locater 12 determines the locations of Molecule B with respect to Molecule A in relation to the children generated, in which the locations are according to the six variables.

On the other hand, in a case where G is threshold value (Step S66; Yes), the evaluator 14, returning to FIG. 8, selects a location at which the attractive interaction between Molecule A and Molecule B is large (Step S7). The intermolecular interaction analysis device 1 stores the evaluation information 3 in the main memory 5 and the external memory 6, and terminates the three-dimensional structure prediction processing of a complex.

An intermolecular interaction analysis device 1 according to the present embodiment evaluates an attractive interaction between Molecule A and Molecule B on the basis of electrostatic complementarity. The attractive interaction significantly contributes to the stability of binding between Molecule A and Molecule B in the formation of a complex of Molecule A and Molecule B, thus making it possible to predict the three-dimensional structure of a complex of Molecule A and Molecule B with high accuracy. An attractive interaction predicted by the intermolecular interaction analysis device 1 can also be used as a docking score in the structure prediction of a complex of Molecule A and Molecule B. In addition, the intermolecular interaction analysis device 1 can be used to detect an unknown protein-protein interaction. Furthermore, using an antibody as Molecule A and an antigen as Molecule B enables the intermolecular interaction analysis device 1 to be used to predict an epitope to which the antibody binds, and thus, the device can contribute to development of an antibody drug, and the like, and control the cost of development of an antibody drug.

In this regard, in the present embodiment, the intermolecular interaction analysis device 1 uses the electron density data 23, electron density data 24, electrostatic potential data 25, and electrostatic potential data 26 stored as the input data 2 in the external memory 6, but the intermolecular interaction analysis device 1 may include a computer that computes the electron density and electrostatic potential of Molecule A in relation to the three-dimensional structure data 21 on Molecule A, and computes the electron density and electrostatic potential of Molecule B in relation to the three-dimensional structure data 22 on Molecule B. In this case, the intermolecular interaction analysis device 1 stores, in the external memory 6, an FMO-method computing program that performs an FMO method on the three-dimensional structure data 21 and the three-dimensional structure data 22, and the computer performs the FMO-method computing program to compute the electron density and electrostatic potential of Molecule A and the electron density and electrostatic potential of Molecule B.

In this regard, the molecule locater 12 is set to repeat determination of a plurality of locations of Molecule B with respect to the position of Molecule A until G becomes a threshold value, but the molecule locater is not limited to this. The molecule locater 12 may repeat determination of a plurality of locations of Molecule B with respect to the position of Molecule A in accordance with a genetic algorithm until the location of Molecule B, having an attractive interaction larger than a threshold value in size, is obtained.

The above-described hardware configuration and software configuration of the intermolecular interaction analysis device 1 are each one example, and can be changed or modified in any way.

The central portion that performs the processing of the intermolecular interaction analysis device 1 including the CPU 4, main memory 5, external memory 6, operation unit 7, display 8, communication interface 9, internal bus 10, and the like can be produced using a usual computer system, without relying on a special system. The intermolecular interaction analysis device 1 that performs the above-described processing may be configured, for example, by storing a computer program for the performance of the above-described operation in a computer-readable storage medium (flexible disk, compact disc read only memory (CD-ROM), digital versatile disk read only memory (DVD-ROM), or the like), distributing the medium, and installing the computer program in a computer. Alternatively, the intermolecular interaction analysis device 1 may be configured by storing the computer program in a storage device included in a server device on a communication network, such as the internet, and, for example, allowing a usual computer system to download the computer program.

For example, in a case where the function of the intermolecular interaction analysis device 1 is achieved through operation divided between an operating system (OS) and an application program or through cooperation between an OS and an application program, only the application program portion may be stored in a storage medium or a storage device.

The computer program may be superimposed on a carrier wave, and delivered via a communication network. For example, a computer program may be posted on a bulletin board system (BBS) on a communication network, and the computer program may be delivered via a network. Then, this computer program may be started up, and performed under the control of an OS in the same manner as another application program, thereby enabling the processing to be performed. Such a configuration is possible.

The present disclosure is more specifically described with reference to the following Examples, and the present disclosure is not limited to the Examples.

Examples

The performance of the three-dimensional structure prediction of a complex of Molecule A and Molecule B by an intermolecular interaction analysis device 1 according to the above-described embodiment was studied using 20 kinds of protein complexes for benchmarking. For the 20 kinds of protein complexes, reference was made to Thom Vreven and 11 others, Updates to the integrated protein-protein interaction benchmarks: Docking benchmark version 5 and affinity benchmark version 2, J Mol Biol, 427, 19, 2015, 3031-3041. Two single three-dimensional structures were tested in the respective cases: a case where a bound three-dimensional structure was utilized; and a case where an unbound three-dimensional structure was utilized.

First, three-dimensional structure data on two proteins the three-dimensional structure of a complex of which was desired to be predicted was obtained from PDB. Here, a protein to be fixed was used as a receptor (Molecule A), and a protein to be located with respect to the receptor was used as a ligand (Molecule B). Next, the electron density and electrostatic potential of each protein were computed with grid cubes using the FMO method, and stored as grid data. The length of a side of the grid cube was set at 0.3 Å. The coordinates of each protein and the coordinates of the grid cube were moved to the origin, and preparations were made so that structure prediction computation could start from the origin. The ligand moved to the origin was located at various positions around the receptor, and the attractive interaction on the contact face was evaluated from the grid data stored.

In the translation of a ligand, a value between -60.0 Å and 60.0 Å was randomly outputted for each of the vectors in the x-axis direction, y-axis direction, and z-axis direction, and the maximum value of the size of the vector was set to 60 Å. As each of three variables related to the rotation, a value between -1.0 and 1.0 was randomly outputted for each of the vectors in the x-axis direction, y-axis direction, and z-axis direction, and the maximum value of the size of the vector was set to 1. In addition, the rotation angle was a value obtained by multiplying, by 360 degrees, the size of the rotation vector outputted.

The location of the ligand was determined with six variables of the translation and the rotation. As an optimization problem of six parameters, a search was made through a genetic algorithm. First, 200 initial groups of six variables were randomly generated. For the locations of the ligand, in which the locations corresponded to six variables generated, contact faces were defined, and the attractive interactions were evaluated. The complexes were ranked in the order of magnitude of the attractive interaction, and six variables as parents were selected from the higher-ranked complexes. After the ranking, the size of a difference between each variable and the next variable in the ranking was computed among the six variables. In a case where the size of the difference between the variables related to the translation was smaller than 5.0 Å, and where the size of the difference between the variables related to the rotation was smaller than 0.1, the variables were deemed to be duplicate, and one of the variables was deleted.

From the parents selected, combinations were made, and children were generated from the parents made. To the children generated, a mutation was caused with a probability. In addition, 100 sets of six variables are newly made randomly. At this point of time, next-generation groups of individuals were made. For the locations of the ligand, in which the locations corresponded to 200 sets of six variables newly made, contact faces were defined, and the attractive interactions were evaluated. The above-described processing was continued until the number of generations in the computation of the genetic algorithm became a threshold value (50000), whereby a final predicted complex was obtained.

For the crossover, 10 sets were selected from the highest-ranked sets, and two combinations as parents were selected from the 10 sets. In this manner, 45 combinations of parents were made. From the six variables of one parent, three variables to be crossed over were selected. The variables selected were exchanged with the other parent, whereby two children were generated. As a result, 90 children were generated.

As to a mutation, nine of the 90 children generated were set to undergo a mutation. For the children selected as children set to undergo a mutation, either three variables related to the translation or three variables related to the rotation were randomly generated anew. For evaluation of the attractive interaction, the neutralized electrostatic potential and the evaluation function represented by the above-described Formula 18 were used. In addition, as the number of points of the contact face, the number of points at which the value of the electron density was larger than $10^{-4}$ a.u. was used.

Results

The three-dimensional structure of the top-ranked complex of a receptor and a ligand, the complex having the largest attractive interaction, was superposed on the three-dimensional structure obtained through X-ray crystallography obtained from PDB, and a root-mean-square deviation (RMSD) at the atomic position was computed. Generally, in a case where a bound three-dimensional structure is utilized, the prediction is considered easy, and in a case where an unbound three-dimensional structure is utilized, the prediction is considered difficult. In Table 1, the RMSDs of the bound structures and the unbound structures are listed in relation to 20 kinds of complexes.

[Table 1]

| PDB ID of complex | bound RMSD(Å) | RMSD (Å) of three-dimensional structure in agreement at lower rank | unbound RMSD(Å) | PDB ID of receptor | PDB ID ligand | RMSD (Å) of three-dimensional structure in agreement at lower rank |
|---|---|---|---|---|---|---|
| 1JTD | **1.8746** | - | 67.9555 | 3Q10 | 1BTL | 3.8798 (Rank; 56) |
| 1RKE | **1.5431** | - | 24.1891 | 1SYQ | 3MYI | 6.2326 (Rank; 16) |

(continued)

| PDB ID of complex | bound RMSD(Å) | RMSD (Å) of three-dimensional structure in agreement at lower rank | unbound RMSD(Å) | PDB ID of receptor | PDB ID ligand | RMSD (Å) of three-dimensional structure in agreement at lower rank |
|---|---|---|---|---|---|---|
| 2GAF | **2.9874** | - | 18.8818 | 3OWG | 1VPT | - |
| 2VXT | **3.8344** | - | **3.5960** | 2VXU | 1J0S | - |
| 2W9E | **1.5982** | - | 75.1400 | 2W9D | 1QM1 | - |
| 2X9A | **1.2491** | - | 40.2713 | 1S62 | 2X9B | - |
| 3A4S | 50.9861 | 1.7849 (Rank; 2) | 52.3950 | 1A3S | 3A4R | 9.1907 (Rank; 12) |
| 3BIW | **9.5874** | - | 14.7858 | 3BIX | 2R1D | 4.6372 (Rank; 60) |
| 3BX7 | **1.4270** | - | 39.6186 | 3BX8 | 3OSK | - |
| 3F1P | 21.5270 | 2.6815 (Rank; 4) | 23.3282 | 1P97 | 1X0O | - |
| 3G6D | **2.4523** | - | 12.1553 | 3G6A | 1IK0 | 9.6421 (Rank; 22) |
| 3H11 | **1.2571** | - | 58.5118 | 4JJ7 | 3H13 | 8.9960 (Rank; 17) |
| 3H2V | 36.0290 | 2.3920 (Rank; 3) | 41.8649 | 3MYI | 1WI6 | - |
| 3K75 | 61.5862 | 2.0273 (Rank; 2) | 27.0084 | 1BPB | 3K77 | - |
| 3L89 | **1.3526** | - | **3.5249** | 3L88 | 1CKL | - |
| 3S9D | 51.3159 | 1.7433 (Rank; 18) | 58.5716 | 1N6U | 1ITF | - |
| 3V6Z | **3.7904** | - | 69.9319 | 3V6F | 3KXS | - |
| 3VLB | **1.4020** | - | **1.8198** | 3VLA | 3VL8 | - |
| 4DN4 | **2.1694** | - | 28.7753 | 4DN3 | 1DOL | - |
| 4G6J | **1.4474** | - | 25.1381 | 4G5Z | 411B | - |

In a case where RMSD was less than 10 Å in this Example, the predicted structure was deemed to be in agreement with the three-dimensional structure obtained through X-ray crystallography. In the case of the bound structures, the top-ranked three-dimensional structure in each of 15 kinds thereof was in agreement with the three-dimensional structure obtained through X-ray crystallography. In the case of the remaining five kinds of bound structures, the lower-ranked three-dimensional structure was in agreement with the three-dimensional structure obtained through X-ray crystallography. In the case of the unbound structures, the top-ranked three-dimensional structure in each of three kinds thereof was in agreement with the three-dimensional structure obtained through X-ray crystallography. In the case of six kinds out of the remaining 17 kinds of unbound structures, the lower-ranked three-dimensional structure was in agreement with the three-dimensional structure obtained through X-ray crystallography, but in the case of eight kinds, even the lower-ranked structure was not in agreement with the three-dimensional structure obtained through X-ray crystallography.

To summarize, the bound structures found having the top-ranked three-dimensional structure in agreement with the three-dimensional structure obtained through X-ray crystallography accounted for 75%. The unbound structures found having the top-ranked three-dimensional structure in agreement with the three-dimensional structure obtained through X-ray crystallography accounted for 15%. According to Non-Patent Literature 1, such agreement in the bound structures and the unbound structures accounted for 60% and 12% respectively at the highest. The prediction of the three-dimensional structure of a complex according to the present Example has been revealed to have high accuracy.

The foregoing describes some example embodiments for explanatory purposes. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-025282, filed on February 21, 2023, the entire disclosure of which is incorporated by reference herein.

**EP 4 654 206 A1**

Industrial Applicability

The present disclosure is useful for analysis of interaction between proteins.

Reference Signs List

1    Intermolecular interaction analysis device
2    Input data
3    Evaluation information
4    CPU
5    Main memory
6    External memory
7    Operation unit
8    Display
9    Communication interface
10   Internal bus
11   Storage
12   Molecule locater
13   Contact face definer
14   Evaluator
21   Three-dimensional structure data on Molecule A
22   Three-dimensional structure data on Molecule B
23   Electron density data on Molecule A
24   Electron density data on Molecule B
25   Electrostatic potential data on Molecule A
26   Electrostatic potential data on Molecule B
51   Computing program

**Claims**

1.  An intermolecular interaction analysis device comprising: a molecule locater that determines a location of a second molecule with respect to a position of a first molecule,

    wherein the first molecule contains a plurality of amino acid residues bound via a peptide bond, and is indicated by three-dimensional structure data on the first molecule, and wherein the second molecule contains a plurality of amino acid residues bound via a peptide bond, and is indicated by three-dimensional structure data on the second molecule;
    a contact face definer that defines, as a contact face, a face on which an electron density of the first molecule computed from the three-dimensional structure data on the first molecule and an electron density of the second molecule computed from the three-dimensional structure data on the second molecule are equal to each other; and
    an evaluator that evaluates an attractive interaction between the first molecule and the second molecule on the basis of electrostatic complementarity between the first molecule and the second molecule on the contact face, wherein the electrostatic complementarity is quantitated from an electrostatic potential of the first molecule computed from the three-dimensional structure data on the first molecule and an electrostatic potential of the second molecule computed from the three-dimensional structure data on the second molecule.

2.  The intermolecular interaction analysis device according to claim 1,
    wherein the evaluator evaluates the attractive interaction between the first molecule and the second molecule on the basis of an index indicative of an extent of the contact face and on the basis of a magnitude of the electrostatic complementarity between the first molecule and the second molecule.

3.  The intermolecular interaction analysis device according to claim 1 or 2, wherein the contact face definer defines, as the contact face, a face on which the electron density of the first molecule is equal to or more than a reference value.

4.  The intermolecular interaction analysis device according to claim 1 or 2,

wherein the evaluator quantitates the electrostatic complementarity from:

a value obtained by adding, to a value of the electrostatic potential of the first molecule at each position of the contact face, a first constant for bringing, to 0, a total value of the electrostatic potential of the first molecule on the contact face by shifting the whole electrostatic potential of the first molecule on the contact face, and;
a value obtained by adding, to a value of the electrostatic potential of the second molecule at each position of the contact face, a second constant for bringing, to 0, a total value of the electrostatic potential of the second molecule on the contact face by shifting the whole electrostatic potential of the second molecule on the contact face.

5. The intermolecular interaction analysis device according to claim 1 or 2,

wherein the molecule locater determines a plurality of locations of the second molecule with respect to the position of the first molecule in accordance with translation of the second molecule in a coordinate system having an x axis, a y axis, and a z axis perpendicular to one other and rotation of the second molecule about the x axis, the y axis, and the z axis as axes of rotation,
wherein the contact face definer defines the contact face for each of the plurality of locations of the second molecule, and
wherein the evaluator evaluates the attractive interaction between the first molecule and the second molecule on each of the contact faces.

6. The intermolecular interaction analysis device according to claim 5,

wherein the molecule locater further determines a plurality of locations of the second molecule with respect to the position of the first molecule in accordance with translation of the second molecule in a coordinate system having the x axis, the y axis, and the z axis and rotation of the second molecule about the x axis, the y axis, and the z axis as axes of rotation, using a genetic algorithm, on the basis of a size of the attractive interaction between the first molecule and the second molecule on each of the contact faces evaluated by the evaluator,
wherein the contact face definer defines the contact face for each of the plurality of locations of the second molecule, and
wherein the evaluator evaluates the attractive interaction between the first molecule and the second molecule on each of the contact faces.

7. The intermolecular interaction analysis device according to claim 6,
wherein the evaluator selects, from the plurality of locations of the second molecule, a location having a large attractive interaction between the first molecule and the second molecule.

8. The intermolecular interaction analysis device according to claim 1 or 2,
wherein the electron density and electrostatic potential of the first molecule and the electron density and electrostatic potential of the second molecule are computed using a fragment molecular orbital method.

9. An intermolecular interaction analysis method comprising:

determining a location of a second molecule with respect to a position of a first molecule,

wherein the first molecule contains a plurality of amino acid residues bound via a peptide bond, and is indicated by three-dimensional structure data on the first molecule, and
wherein the second molecule contains a plurality of amino acid residues bound via a peptide bond, and is indicated by three-dimensional structure data on the second molecule;

defining, as a contact face, a face on which an electron density of the first molecule computed from the three-dimensional structure data on the first molecule and an electron density of the second molecule computed from the three-dimensional structure data on the second molecule are equal to each other; and
evaluating an attractive interaction between the first molecule and the second molecule on the basis of electrostatic complementarity between the first molecule and the second molecule on the contact face, wherein the electrostatic complementarity is quantitated from an electrostatic potential of the first molecule computed from the three-dimensional structure data on the first molecule and an electrostatic potential of the second molecule computed from the three-dimensional structure data on the second molecule.

10. A program that allows a computer to function as:

a molecule locater that determines a location of a second molecule with respect to a position of a first molecule,

wherein the first molecule contains a plurality of amino acid residues bound via a peptide bond, and is indicated by three-dimensional structure data on the first molecule, and
wherein the second molecule contains a plurality of amino acid residues bound via a peptide bond, and is indicated by three-dimensional structure data on the second molecule;

a contact face definer that defines, as a contact face, a face on which an electron density of the first molecule computed from the three-dimensional structure data on the first molecule and an electron density of the second molecule computed from the three-dimensional structure data on the second molecule are equal to each other; and
an evaluator that evaluates an attractive interaction between the first molecule and the second molecule on the basis of electrostatic complementarity between the first molecule and the second molecule on the contact face, wherein the electrostatic complementarity is quantitated from an electrostatic potential of the first molecule computed from the three-dimensional structure data on the first molecule and an electrostatic potential of the second molecule computed from the three-dimensional structure data on the second molecule.

# FIG. 1

## FIG. 2

# FIG. 3A

# FIG. 3B

# FIG. 4

# FIG. 5

# FIG. 6

EP 4 654 206 A1

# FIG. 7

```
┌─────────────────────────────────────────┐
│   ATTRACTIVE INTERACTION EVALUATION      │
│              PROCESSING                   │
└─────────────────────────────────────────┘
                    │
                    ▼
┌─────────────────────────────────────────┐  S1
│  READ-IN OF THREE-DIMENSIONAL STRUCTURE  │
│           DATA AND THE LIKE              │
└─────────────────────────────────────────┘
                    │
                    ▼
┌─────────────────────────────────────────┐  S2
│   DETERMINATION OF LOCATION OF MOLECULE B │
└─────────────────────────────────────────┘
                    │
                    ▼
┌─────────────────────────────────────────┐  S3
│        DEFINITION OF CONTACT FACE        │
└─────────────────────────────────────────┘
                    │
                    ▼
┌─────────────────────────────────────────┐  S4
│   EVALUATION OF ATTRACTIVE INTERACTION   │
└─────────────────────────────────────────┘
                    │
                    ▼
┌─────────────────────────────────────────┐
│                  END                     │
└─────────────────────────────────────────┘
```

# FIG. 8

```
┌─────────────────────────────────────────────┐
│  THREE-DIMENSIONAL STRUCTURE PREDICTION      │
│  PROCESSING OF COMPLEX                        │
└─────────────────────────────────────────────┘
                      │
                      ▼
┌─────────────────────────────────────────────┐  S5
│  READ-IN OF THREE-DIMENSIONAL STRUCTURE DATA │
│  AND THE LIKE                                 │
└─────────────────────────────────────────────┘
                      │
                      ▼
┌─────────────────────────────────────────────┐  S6
│  SEARCH PROCESSING BY GENETIC ALGORITHM      │
└─────────────────────────────────────────────┘
                      │
                      ▼
┌─────────────────────────────────────────────┐  S7
│  SELECTION OF LOCATION HAVING                │
│  LARGE ATTRACTIVE INTERACTION               │
└─────────────────────────────────────────────┘
                      │
                      ▼
┌─────────────────────────────────────────────┐
│                   END                         │
└─────────────────────────────────────────────┘
```

# FIG. 9

SEARCH PROCESSING BY
GENETIC ALGORITHM

GENERATION OF $N_1$ NUMBER
OF INITIAL GROUPS — S61

DETERMINATION OF
LOCATION OF MOLECULE B — S62

DEFINITION OF CONTACT
FACE — S63

EVALUATION OF
ATTRACTIVE INTERACTION — S64

$G \leftarrow G + 1$ — S65

S66 — G = THRESHOLD VALUE?

No

Yes

SELECTION OF PARENTS — S67

GENERATION OF $N_2$ NUMBER
OF CHILDREN — S68

MUTATION — S69

GENERATION OF ANOTHER
$N_2$ NUMBER OF CHILDREN — S70

END

26

## INTERNATIONAL SEARCH REPORT

| International application No. |
| --- |
| **PCT/JP2024/005667** |

**A. CLASSIFICATION OF SUBJECT MATTER**

*G16B 15/30*(2019.01)i
FI:   G16B15/30

According to International Patent Classification (IPC) or to both national classification and IPC

**B. FIELDS SEARCHED**

Minimum documentation searched (classification system followed by classification symbols)

G16B15/30

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2006-190234 A (UNIV. WASEDA) 20 July 2006 (2006-07-20)<br>entire text, all drawings | 1-10 |
| A | JP 2008-052308 A (UNIV. WASEDA) 06 March 2008 (2008-03-06)<br>entire text, all drawings | 1-10 |

☐ Further documents are listed in the continuation of Box C.    ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **14 March 2024** | **26 March 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/005667**

| Patent document cited in search report | | | Publication date (day/month/year) | Patent family member(s) | Publication date (day/month/year) |
|---|---|---|---|---|---|
| JP | 2006-190234 | A | 20 July 2006 | (Family: none) | |
| JP | 2008-052308 | A | 06 March 2008 | WO 2006/062095 A1 | |

Form PCT/ISA/210 (patent family annex) (July 2022)

## REFERENCES CITED IN THE DESCRIPTION

**Patent documents cited in the description**

- JP 2008135019 A

- JP 2023025282 A

**Non-patent literature cited in the description**

- **SHENG-YOUHUANG**. Exploring the potential of global protein-protein docking: an overview and critical assessment of current programs for automatic ab initio docking. *Drug Discovery Today*, 2015, vol. 20 (8), 969-977

- *J Mol Biol*, 2015, vol. 427 (19), 3031-3041